(19) Europäisches Patentamt
European Patent Office
Office européen des brevets

(11) Veröffentlichungsnummer: **0 141 777**
**A2**

(12) # EUROPÄISCHE PATENTANMELDUNG

(21) Anmeldenummer: **84810429.5**

(22) Anmeldetag: **03.09.84**

(51) Int. Cl.⁴: **C 07 D 417/12**, C 07 D 403/12, C 07 D 401/12, C 07 D 239/46, C 07 D 251/16, C 07 D 413/12, C 07 D 405/12, C 07 D 239/42, A 01 N 47/36
// C07D277/10, C07D239/26, C07D233/54, C07D249/08, C07D213/32, C07D239/36

(30) Priorität: **09.09.83 CH 4932/83**

(43) Veröffentlichungstag der Anmeldung: **15.05.85**
**Patentblatt 85/20**

(84) Benannte Vertragsstaaten: **BE CH DE FR GB IT LI**

(71) Anmelder: **CIBA-GEIGY AG, Postfach, CH-4002 Basel (CH)**

(72) Erfinder: **Töpfl, Werner, Dr., Dorneckstrasse 68, CH-4143 Dornach (CH)**
Erfinder: **Pissiotas, Georg, Dr., Am Sonnenrain 71, D-7850 Lörrach (DE)**

(54) **Herbizide Sulfonylharnstoffe.**

(57) Substituierte N-Phenylsulfonyl-N′-pyrimidinyl- und -triazinylharnstoffe der allgemeinen Formel

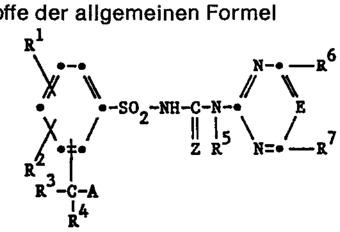

und die Salze dieser Verbindungen mit Aminen, Alkali- oder Erdalkalimetallbasen oder mit quaternären Ammoniumbasen haben gute pre- und postemergent-selektive herbizide und wuchsregulierende Eigenschaften.

In dieser Formel bedeuten

$R^1$ Wasserstoff, Halogen, Nitro, Cyan, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfonyl, $C_1$-$C_4$-Alkylsulfinyl, $-CO-R^8$, $-NR^9R^{10}$, $-CO-NR^{11}R^{12}$ oder $-SO_2-NR^{13}R^{14}$,

$R^2$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^3$ Wasserstoff, $C_1$-$C_4$-Alkyl, $C_1$-$C_4$-Halogenalkyl oder Cyan,

$R^4$ Wasserstoff oder $C_1$-$C_4$-Alkyl,

$R^5$ Wasserstoff, $C_1$-$C_4$-Alkyl, $C_1$-$C_4$-Alkoxy,

$R^6$ und $R^7$ unabhängig voneinander Wasserstoff, Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_1$-$C_4$-Halogenalkylthio, $C_2$-$C_4$-Alkoxyalkyl, $C_3$-$C_6$-Cycloalkyl oder $-NR^{15}R^{16}$,

$R^8$ Wasserstoff, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_2$-$C_4$-Alkoxyalkoxy,

$R^9$, $R^{10}$, $R^{11}$, $R^{12}$, $R^{13}$, $R^{14}$, $R^{15}$ und $R^{16}$ unabhängig voneinander Wasserstoff oder $C_1$-$C_4$-Alkyl,

A einen Rest $-Y-(CH_2)_n-R^{17}$ oder

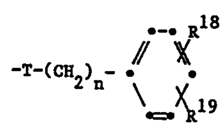

$R^{17}$ einen 5- bis 6gliedrigen ungesättigen heterocyclischen Rest,

$R^{18}$ und $R^{19}$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, Nitro oder Cyan

E Sticksroff oder $-CH=$,

Y Sauerstoff, Schwefel oder eine direkte Bindung,

T und Z unabhängig voneinander Sauerstoff oder Schwefel und

n Null oder eins.

**EP 0 141 777 A2**

CIBA-GEIGY AG
Basel (Schweiz)

5-14578/=

Herbizide Sulfonylharnstoffe

Die vorliegende Erfindung betrifft neue, herbizid wirksame und pflanzenwuchsregulierende substituierte N-Phenylsulfonyl-N'-pyridimidinyl- und triazinyl-harnstoffe, Verfahren zu ihrer Herstellung, sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums. Darüberhinaus betrifft die Erfindung auch als Zwischenprodukte hergestellte neue substituierte Phenylsulfonamide.

Die erfindungsgemässen substituierten N-Phenylsulfonyl-N'-pyrimidinyl- und -triazinyl-harnstoffe entsprechen der Formel I

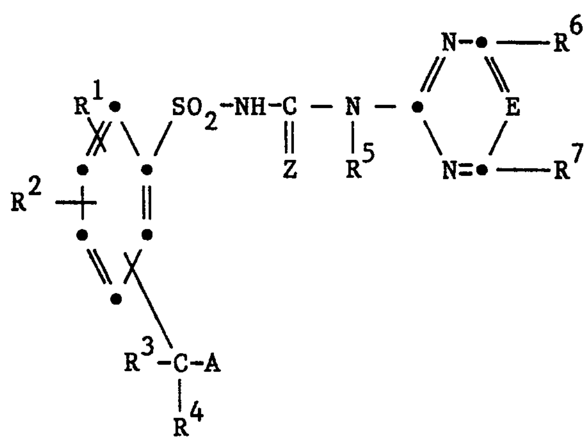

(I),

worin

$R^1$ Wasserstoff, Halogen, Nitro, Cyan, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfonyl, $C_1$-$C_4$-Alkylsulfinyl, $-CO-R^8$, $-NR^9R^{10}$, $-CO-NR^{11}R^{12}$ oder $-SO_2-NR^{13}R^{14}$,

$R^2$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^3$ Wasserstoff, $C_1$-$C_4$-Alkyl, $C_1$-$C_4$-Halogenalkyl oder Cyan,

$R^4$ Wasserstoff oder $C_1$-$C_4$-Alkyl,

$R^5$ Wasserstoff, $C_1$-$C_4$-Alkyl oder $C_1$-$C_4$-Alkoxy,

$R^6$ und $R^7$ unabhängig voneinander Wasserstoff, Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_1$-$C_4$-Halogenalkylthio, $C_2$-$C_4$-Alkoxyalkyl, $C_3$-$C_6$-Cycloalkyl oder $-NR^{15}R^{16}$,

$R^8$ Wasserstoff, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_2$-$C_4$-Alkoxyalkoxy,

$R^9$, $R^{10}$, $R^{11}$, $R^{12}$, $R^{13}$, $R^{14}$, $R^{15}$ und $R^{16}$ unabhängig voneinander Wasserstoff oder $C_1$-$C_4$-Alkyl,

A einen Rest $-Y-(CH_2)_n-R^{17}$ oder

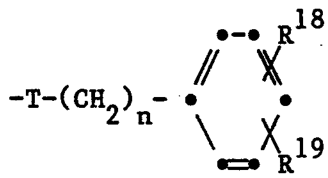

$R^{17}$ einen 5- bis 6-gliedrigen ungesättigen heterocyclischen Rest,

$R^{18}$ und $R^{19}$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, Nitro oder Cyan,

E Stickstoff oder -CH=,

Y Sauerstoff, Schwefel oder eine direkte Bindung,

T und Z unabhängig voneinander Sauerstoff oder Schwefel und

n Null oder eins bedeuten,

sowie den Salzen dieser Verbindungen.

Harnstoffverbindungen, Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den europäischen Patentanmeldungen 44211, 44807 und 44808 sowie den britischen Patentanmeldungen 2 112 783 und 2 112 784 beschrieben.

In den Definitionen ist unter Alkyl geradkettiges oder verzweigtes Alkyl zu verstehen; z.B.: Methyl, Aethyl, n-Propyl, i-Propyl oder die vier isomeren Butyl.

Unter Alkoxy ist zu verstehen: Methoxy, Aethoxy, n-Propyloxy, i-Propyloxy, die vier isomeren Butyloxyreste, n-Amyloxy, i-Amyloxy, 2-Amyloxy oder 3-Amyloxy, insbesondere aber Methoxy, Aethoxy oder i-Propyloxy.

Beispiele für Alkylthio sind Methylthio, Aethylthio, n-Propylthio, i-Propylthio, n-Butylthio oder n-Pentylthio insbesondere aber Methylthio und Aethylthio.

Beispiele für Alkylsulfinyl sind Methylsulfinyl, Aethylsulfinyl, n-Propylsulfinyl und n-Butylsulfinyl, insbesondere aber Methylsulfinyl und Aethylsulfinyl.

Beispiele für Alkylsulfonyl sind Methylsulfonyl, Aethylsulfonyl oder n-Propylsulfonyl, insbesondere aber Methylsulfonyl und Aethylsulfonyl.

Unter Halogen selbst in den Definitionen sowie Halogen als Teil in Halogenalkoxy, Halogenalkyl oder Halogenalkylthio sind Fluor, Chlor und Brom, vorzugsweise jedoch Fluor und Chlor zu verstehen.

Die unter $R^{17}$ definierten ungesättigten 5- bis 6-gliedrigen Heterocyclen umfassen im Rahmen der vorliegenden Erfindung beispielsweise folgende grundlgegende Ringsysteme (L steht für Wasserstoff oder $C_1$-$C_4$-Alkyl):

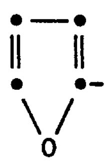
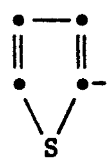
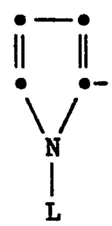

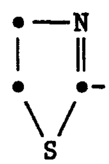
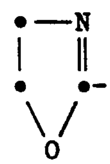
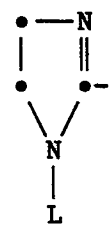

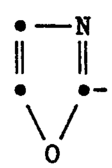
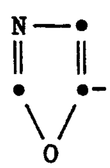
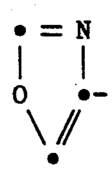

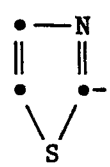
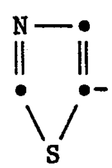
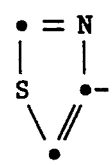

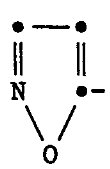
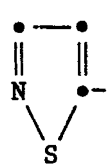
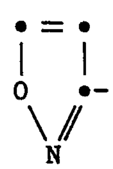

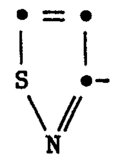
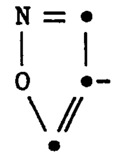
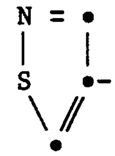
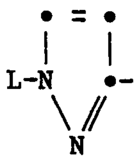
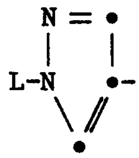
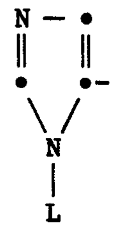
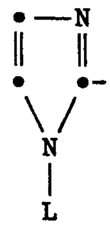
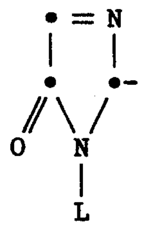
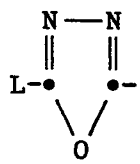
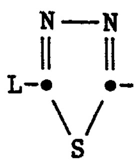
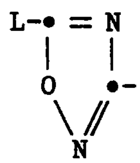
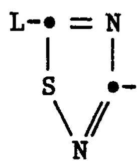
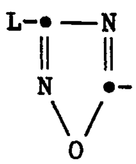
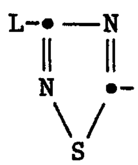
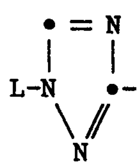
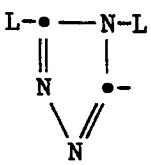
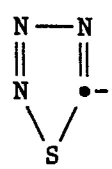
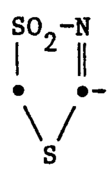
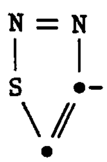
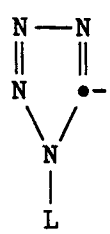
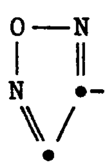

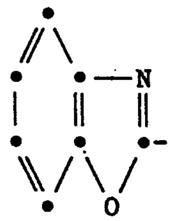

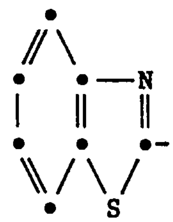

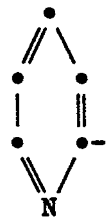

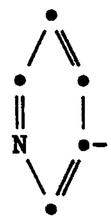

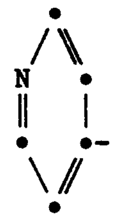

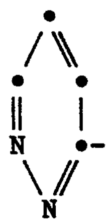

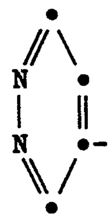

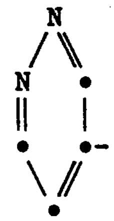

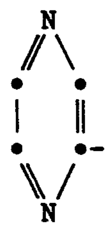

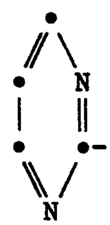

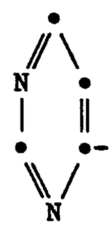

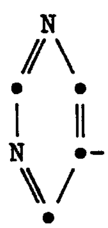

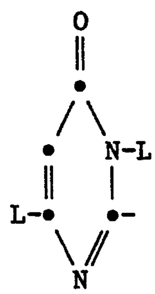

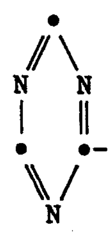

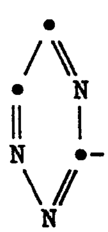

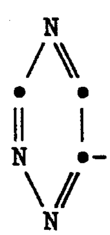

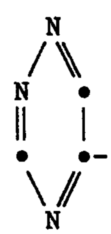

Die von der Definition $R^{17}$ umfassten Hetercyclen sind sämtlich über ein Ringkohlenstoffatom mit dem Grundgerüst des Molekülteils A verknüpft. Ferner sind von der Definition auch die teilhydrierten oder/und teiloxidierten Abkömmlinge eingeschlossen.

Bevorzugte Heterocyclen unter der Definition von $R^{17}$ sind:

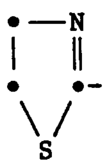 , 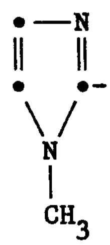 , 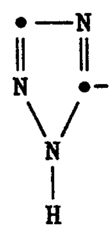 , 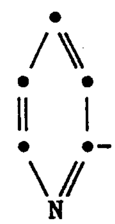 ,

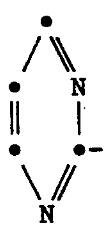 und 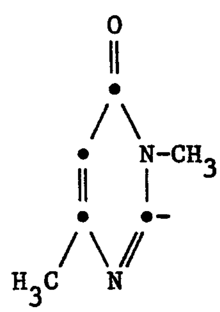

Alkoxyalkylreste werden repräsentiert durch Methoxymethyl, Aethoxymethyl, Methoxyäthyl und Aethoxyäthyl, insbesondere aber Methoxyäthyl, Alkoxyalkoxyreste sind im Rahmen der vorliegenden Erfindung Methoxymethoxy, Alkoxymethoxy, Methoxyäthoxy und Aethoxyäthoxy. Halogenalkyl als Substituent selbst oder als Teil eines anderen Substituenten wie Halogenalkoxy oder Halogenalkylthio steht in der Regel für Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin, Diäthanolamin und 1,4-Diazabicyclo[2.2.2]octan.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Unter den Verbindungen der Formel I sind diejenigen bevorzugt, in denen entweder

a) Z Sauerstoff ist oder
b) $R^1$ Wasserstoff ist oder
c) $R^2$ Wasserstoff ist oder
d) $R^3$ Wasserstoff ist oder
e) $R^4$ Wasserstoff ist oder
f) $R^5$ Wasserstoff ist oder
g) $R^6$ und $R^7$ unabhängig voneinander für Halogen, $C_1$-$C_4$-Alkyl, $C_1$-$C_4$-Alkoxy, Di-$C_1$-$C_4$-alkylamino oder $C_1$-$C_4$-Halogenalkoxy stehen und zusammen höchstens 4 Kohlenstoffatome enthalten oder
h) A für die Gruppe $-Y-(CH_2)_n-R^{17}$ steht.

Unter den Verbindungen der Untergruppe h) sind besonders solche Verbindungen bevorzugt, in denen n für Null steht, insbesondere solche Verbindungen, in denen A für den Rest $-S-R^{17}$ steht. Beispiele für ganz besonders bevorzugte Reste $-S-R^{17}$ sind 4,5-Dihydrothiazol-2-ylthio, 3-Methylimidazol-2-ylthio, 1,3,4-Triazol-2-ylthio, Pyridin-2-ylthio, Pyrimidin-2-ylthio oder 3H-2,6-Dimethyl-3-oxo-pyrimidin-2-ylthio.

Als weitere hervorzuhebende Untergruppen von Verbindungen der Formel I sind diejenigen zu nennen, in denen Z für Sauerstoff steht, $R^1$, $R^2$, $R^3$, $R^4$ und $R^5$ Wasserstoff bedeuten und $R^6$ und $R^7$ unabhängig voneinander für Halogen, $C_1$-$C_4$-Alkyl, $C_1$-$C_4$-Alkoxy, Di-$C_1$-$C_4$-alkylamino oder $C_1$-$C_4$-Halogenalkoxy stehen und zusammen höchstens 4 Kohlenstoffatome enthalten, und besonders diejenigen in denen Z für Sauerstoff steht, $R^1$, $R^2$, $R^3$, $R^4$ und $R^5$ Wasserstoff bedeuten, $R^6$ und $R^7$ unabhängig voneinander für Halogen, $C_1$-$C_4$-Alkyl, $C_1$-$C_4$-Alkoxy, Di-$C_1$-$C_4$-alkylamino oder $C_1$-$C_4$-Halogenalkoxy stehen und zusammen höchstens 4 Kohlenstoffatome enthalten und A für den Rest $-S-R^{17}$ steht und hiervon insbesondere solche, in denen Z für Sauerstoff steht, $R^1$, $R^2$, $R^3$, $R^4$ und $R^5$ Wasserstoff bedeuten, $R^6$ und $R^7$ unabhängig voneinander für Halogen, $C_1$-$C_4$-Alkyl, $C_1$-$C_4$-Alkoxy, Di-$C_1$-$C_4$-alkylamino oder $C_1$-$C_4$-Halogenalkoxy stehen und zusammen höchstens 4 Kohlenstoffatome enthalten und A für einen Rest steht, ausgewählt aus der Gruppe 4,5-Dihydrothiazol-2-ylthio, 3-Methylimidazol-2-ylthio, 1,3,4-Triazol-2-ylthio, Pyridin-2-ylthio, Pyrimidin-2-ylthio oder 3H-2,6-Dimethyl-3-oxopyrimidin-2-ylthio.

Als bevorzugte Einzelsubstanzen sind zu nennen:

N-[2-(4,5-Dihydrothiazol-2-ylthiomethyl)-phenylsulfonyl]-N'-(4-methoxy-6-methyl-pyrimidin-2-yl)harnstoff,
N-[2-(Pyridin-2-ylthiomethyl)-phenylsulfonyl]-N'-(4-methoxy-6-methyl-1.3.5-triazin-2-yl)-harnstoff und
N-[2-(Pyrimidin-2-ylthiomethyl)-phenylsulfonyl]-N'-(4-methoxy-6-methyl-pyrimidin-2-yl)-harnstoff.

Die Herstellung der Verbindungen der Formel I erfolgt im allgemeinen nach den folgenden Methoden.

Nach einem ersten Verfahren werden die Verbindungen der Formel I erhalten, indem man ein substituiertes Phenylsulfonamid der Formel II

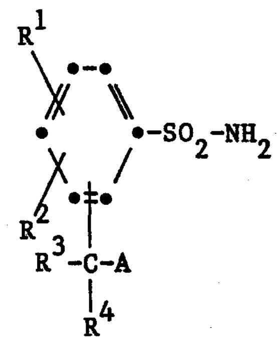

(II),

worin $R^1$, $R^2$, $R^3$, $R^4$, und A die unter Formel I gegebene Bedeutung haben, in Gegenwart einer Base mit einem N-Pyrimidinyl-oder -triazinylcarbamat der Formel III

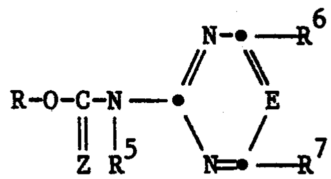

(III),

worin E, $R^5$, $R^6$, $R^7$ und Z die unter Formel I gegebene Bedeutung haben und R für Phenyl, Alkyl oder substituiertes Phenyl steht, umsetzt.

Nach einem zweiten Verfahren gelangt man zu Verbindungen der Formel I, indem man ein substituiertes N-Phenylsulfonylcarbamat der Formel IV

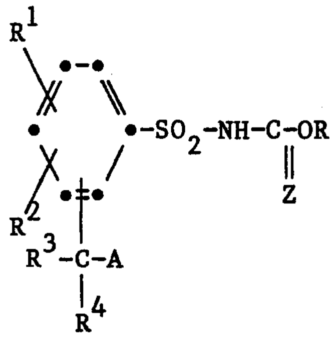

(IV),

worin A, $R^1$, $R^2$, $R^3$, $R^4$, und Z die unter Formel I gegebene Bedeutung haben und R für Phenyl, Alkyl oder substituiertes Phenyl steht, mit einem Aminopyrimidin oder -triazin der Formel V

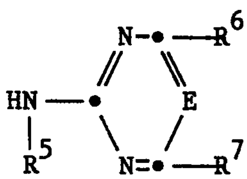

(V),

worin E, $R^5$, $R^6$, und $R^7$ die unter Formel I gegebene Bedeutung haben, umsetzt.

Schliesslich kann man die Verbindungen der Formel I in denen Y keine direkte Bindung bedeutet, auch erhalten, indem man entweder einen Sulfonylharnstoff der Formel VI

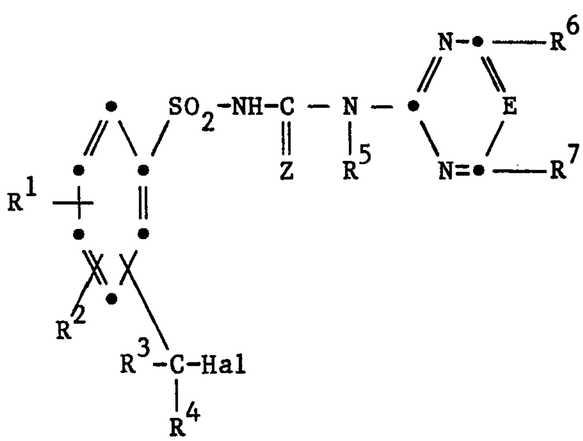

(VI),

worin $R^1$, $R^2$, $R^3$, $R^4$, $R^5$, $R^6$, $R^7$, E und Z die unter Formel I gegebene Bedeutung haben und Hal für Halogen, insbesondere Brom oder Chlor steht, in Gegenwart einer Base mit einem Alkohol oder einem Mercaptan der Formeln VII oder VIII

$H—Y—(CH_2)_n-R^{17}$ , H - T - $(CH_2)_n$ - 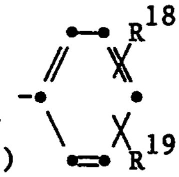

(VII) (VIII)

worin $R^{17}$, $R^{18}$, $R^{19}$, n und T die unter Formel I gegebene Bedeutung haben, und Y für Sauerstoff oder Schwefel steht, umsetzt, oder indem man eine Verbindung der Formel IX

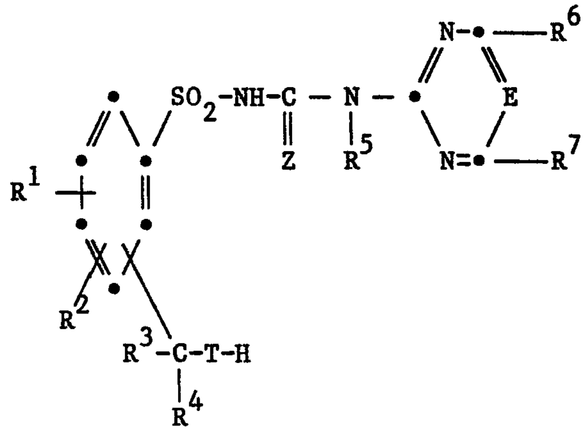

(IX),

worin $R^1$, $R^2$, $R^3$, $R^4$, $R^5$, $R^6$, $R^7$, E, T und Z die unter Formel I gegebene Bedeutung haben, in Gegenwart einer Base mit einer Halogenverbindung der Formeln X oder XI

$Hal—(CH_2)_n—R^{17}$ , Hal-$(CH_2)$ - 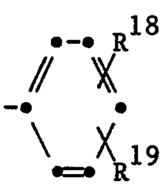

(X) (XI)

worin $R^{17}$, $R^{18}$, $R^{19}$ und n die unter Formel I gegebene Bedeutung haben und Hal für Halogen, vorzugsweise Brom oder Chlor steht, umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, 1,4-Diazabicyclo-(2,2,2)-octan. 1,5-Diazabicyclo(4,3,0)non-5-en oder 1,5-Diazabicyclo(5,4,0)undec-7-en geeignet. Als Basen können aber auch anorganische Basen wie Hydride wie Natrium- oder Calciumhydrid, Hydroxyide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen gereinigt werden.

Die Zwischenprodukte der Formel II sind neu und wurden speziell für die Synthese der Verbindungen der Formel I entwickelt. Sie bilden daher einen Bestandteil der vorliegenden Erfindung.

Die neuen Zwischenprodukte der Formel II werden nach verschiedenen Verfahren hergestellt. So erhält man die Verbindungen der Unterformel IIa und IIb

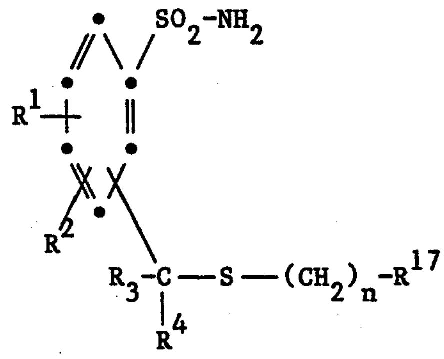

(IIa),

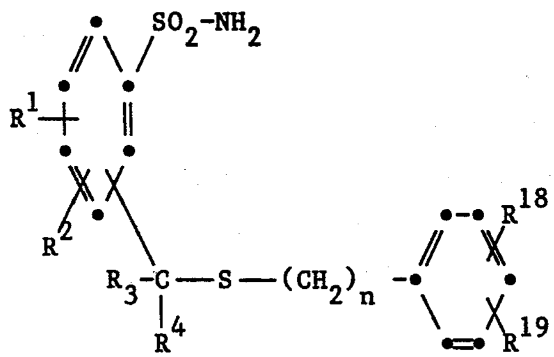

(IIb),

worin $R^1$, $R^2$, $R^3$, $R^4$, $R^{17}$, $R^{18}$, $R^{19}$ und n die unter Formel I gebebene Bedeutung haben, indem man ein Phenylsulfonamid der Formel XII

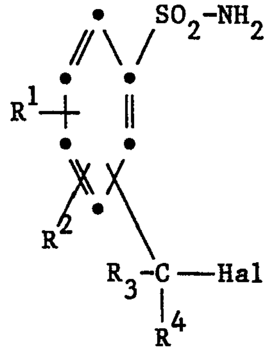

(XII)

worin $R^1$, $R^2$, $R^3$, und $R^4$ die unter Formel I gegebene Bedeutung haben und Hal für Halogen, insbesondere Brom oder Chlor steht, mit einem Mercaptan der Formeln XIII oder XIV

$$H—S—(CH_2)_n—R^{17}$$ ,

(XIII)

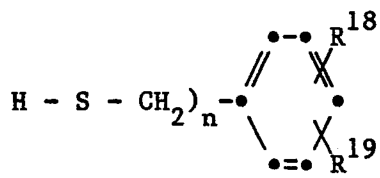

(XIV)

worin $R^{17}$, $R^{18}$, $R^{19}$ und n die unter Formel I gegebene Bedeutung haben, in Gegenwart einer Base umsetzt.

Die Verbindungen der Unterformeln IIc und IId

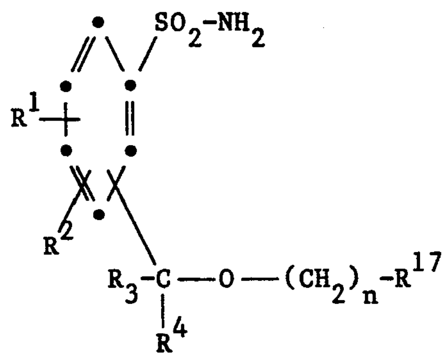

(IIc),

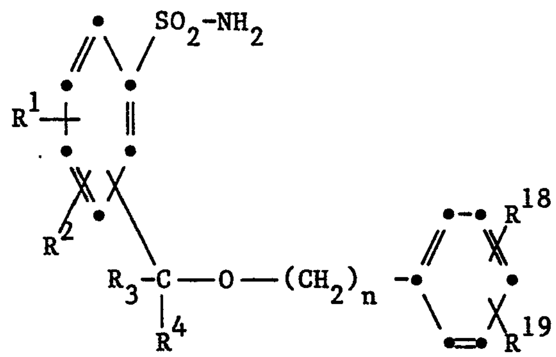

(IId),

worin $R^1$, $R^2$, $R^3$, $R^4$, $R^{17}$, $R^{18}$, $R^{19}$ und n die unter Formel I gegebene Bedeutung haben, indem man entweder

a) eine Verbindung der Formel XV

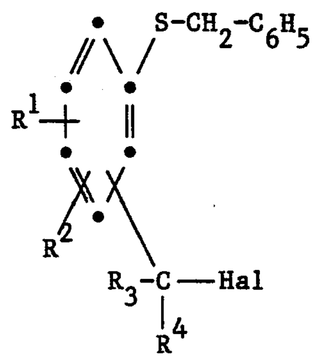

(XV),

worin $R^1$, $R^2$, $R^3$, und $R^4$ die unter Formel I gegebene Bedeutung haben, und Hal für Halogen, insbesondere Brom oder Chlor steht, mit einem Alkohol der Formeln XVI oder XVII

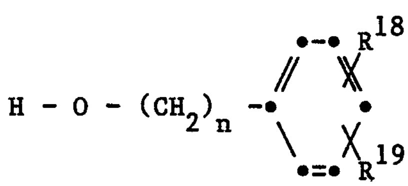

(XVI)

,

$$H-O-(CH_2)_n-R^{17}$$

(XVII)

worin $R^{17}$, $R^{18}$, $R^{19}$ und n die unter Formel I gegebene Bedeutung haben, in Gegenwart einer Base umsetzt oder

b) eine Verbindung der Formel XVIII

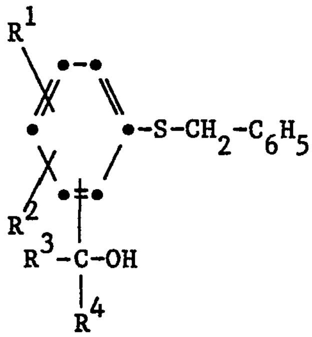

(XVIII),

worin $R^1$, $R^2$, $R^3$, und $R^4$ die unter Formel I gegebene Bedeutung haben, mit einem Halogenid der Formeln XIX oder XX

Hal —$(CH_2)_n$—$R^{17}$ , 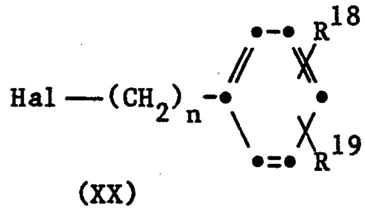

(XIX) (XX)

worin $R^{17}$, $R^{18}$, $R^{19}$ und n die unter Formel I gegebene Bedeutung haben und Hal für Halogen, insbesondere Brom oder Chlor steht, in Gegenwart einer Base umsetzt und das entstandene Zwischenprodukt der Formeln XXI oder XXII

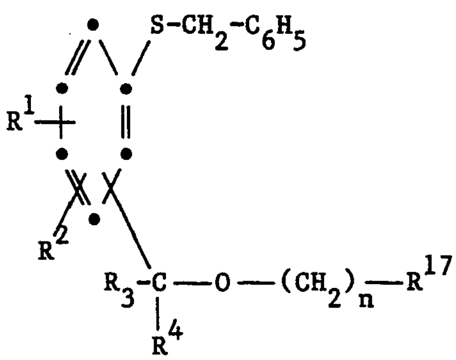

(XXI),

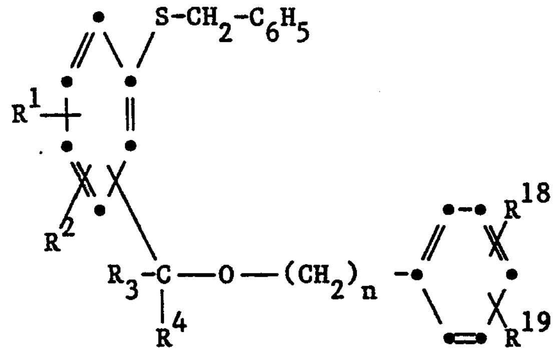

(XXII),

worin $R^1$, $R^2$, $R^3$, $R^4$, A und n die unter Formel I gegebene Bedeutung haben, durch Behandlung mit Chlorgas und anschliessend mit Ammoniak in das Sulfonamid der Formeln IIc oder IId überführt.

Die ebenfalls neuen Sulfonylcarbamate der Formel IV werden durch Umsetzung der Sulfonamide der Formel II mit einem Kohlensäurediester in Gegenwart einer Base erhalten. Aehnliche Verfahren sind in der japanischen Patentschrift 61 169 erwähnt.

Die als Ausgangsmaterialien verwendeten Aminopyrimidine und -triazine der Formel V sowie entsprechende Phenylcarbamate der Formel III sind entweder sei langem bekannt oder in der europäischen Patentanmeldung Nr. 70804 beschrieben oder sie lassen sich nach bekannten Methoden aus dort beschriebenen Verbindungen erhalten.

Die Ausgangsverbindungen der Formel VI sind in der EP-A 44209 beschrieben oder können nach analogem Verfahren erhalten werden.

Die Verbindungen der Formeln VII, VIII, IX, X, XI, XII, XIII, XIV XV, XVI, XVII, XVIII, XIX und XX sind bekannt oder können analog zu bekannten Verfahren hergestellt werden.

Die Verbindungen der Unterformeln IIe

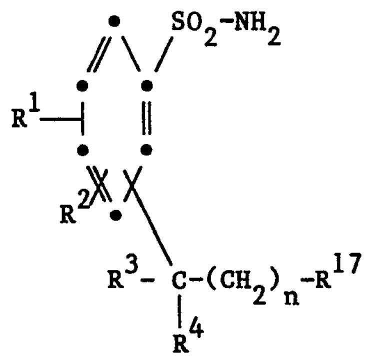

(IIe)

werden nach an sich bekannten Methoden zur Synthese von über Kohlenstoffatome gebundenen Heterocyclen hergestellt.

Beispielhaft seien die folgenden Syntheseschemata zur Herstellung einzelner Beispielverbindungen der Formel IIe skizziert: (L steht für Wasserstoff oder $C_1$-$C_4$-Alkyl).

Schema 1:

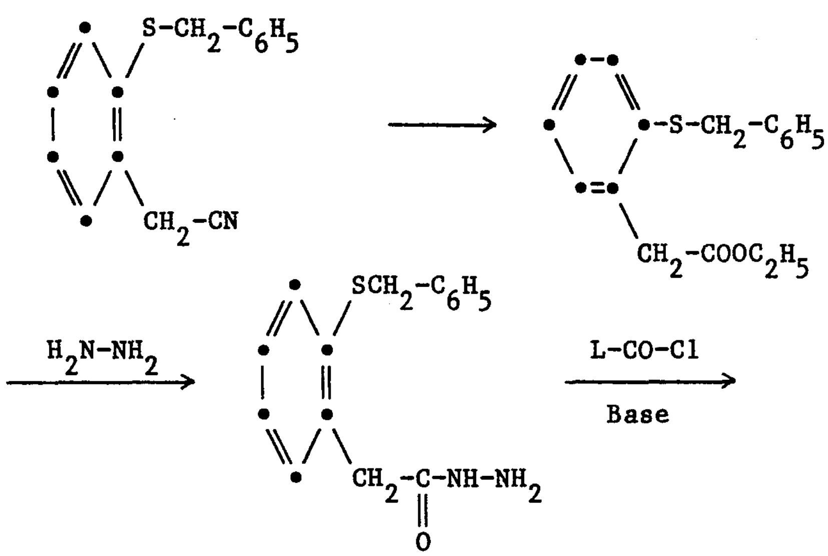

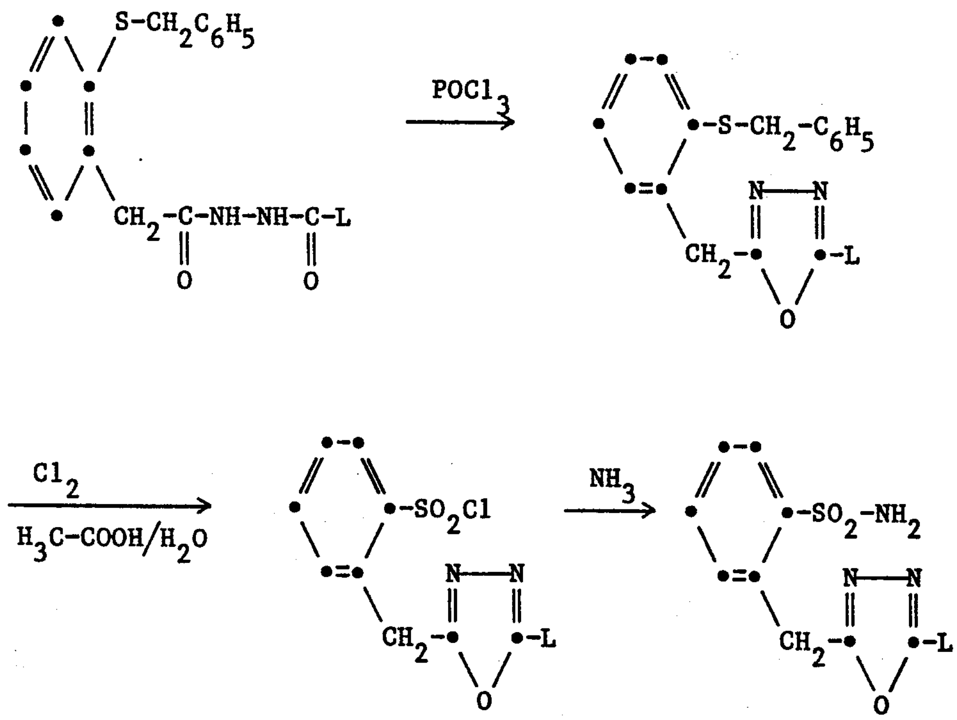
$POCl_3$
$Cl_2$
$H_3C-COOH/H_2O$
$NH_3$

Schema 2:

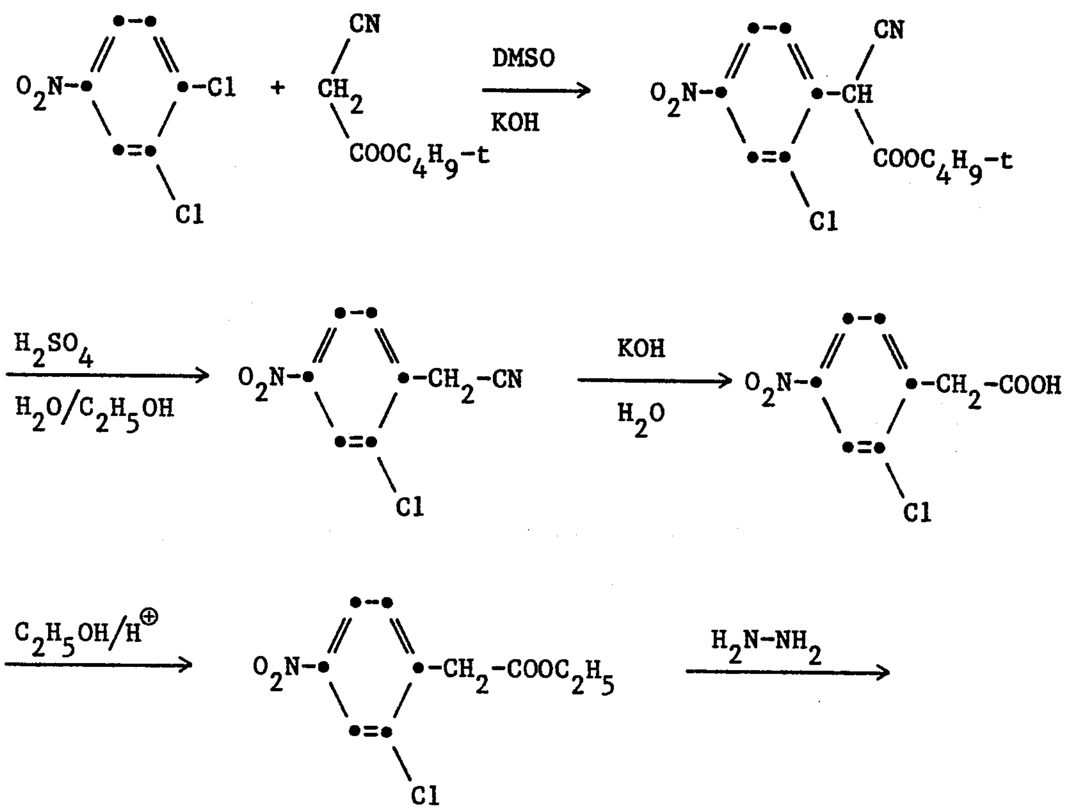
DMSO
KOH
$H_2SO_4$
$H_2O/C_2H_5OH$
KOH
$H_2O$
$C_2H_5OH/H^{\oplus}$
$H_2N-NH_2$

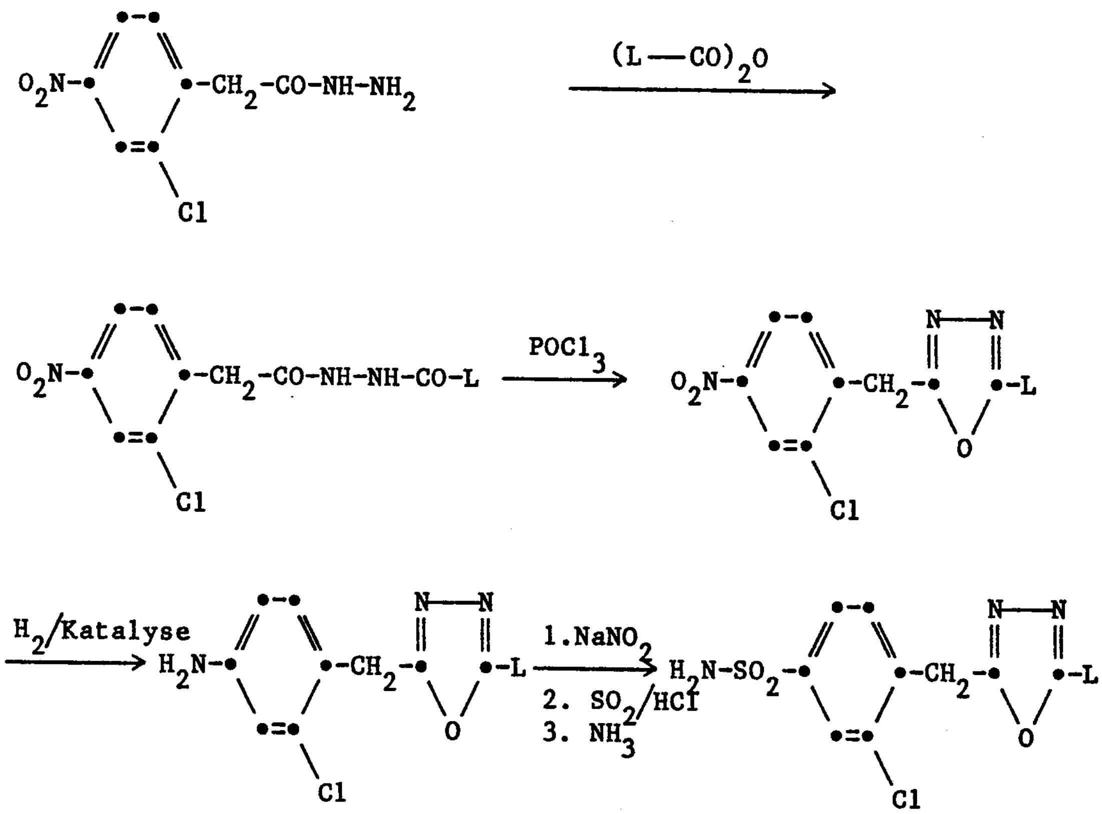
(L—CO)$_2$O
POCl$_3$
H$_2$/Katalyse
1. NaNO$_2$
2. SO$_2$/HCl
3. NH$_3$

Schema 3:

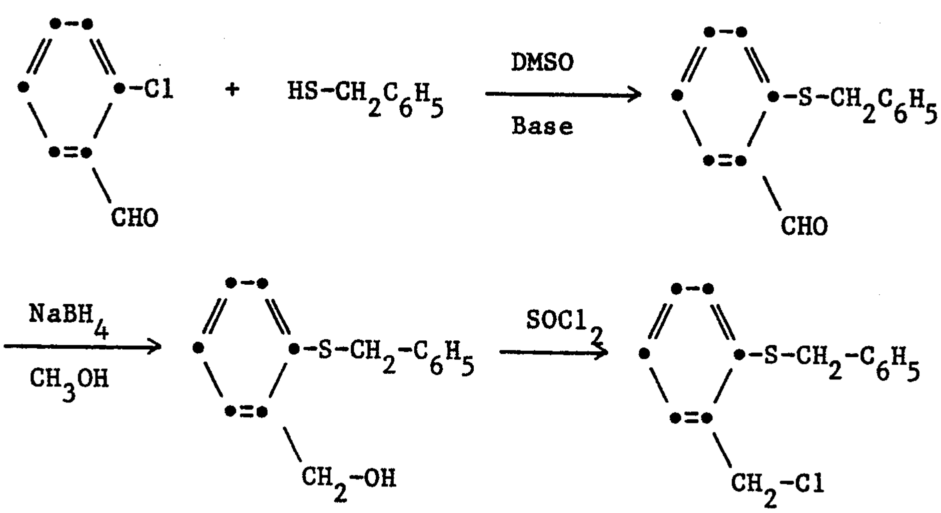
DMSO
Base
NaBH$_4$
CH$_3$OH
SOCl$_2$

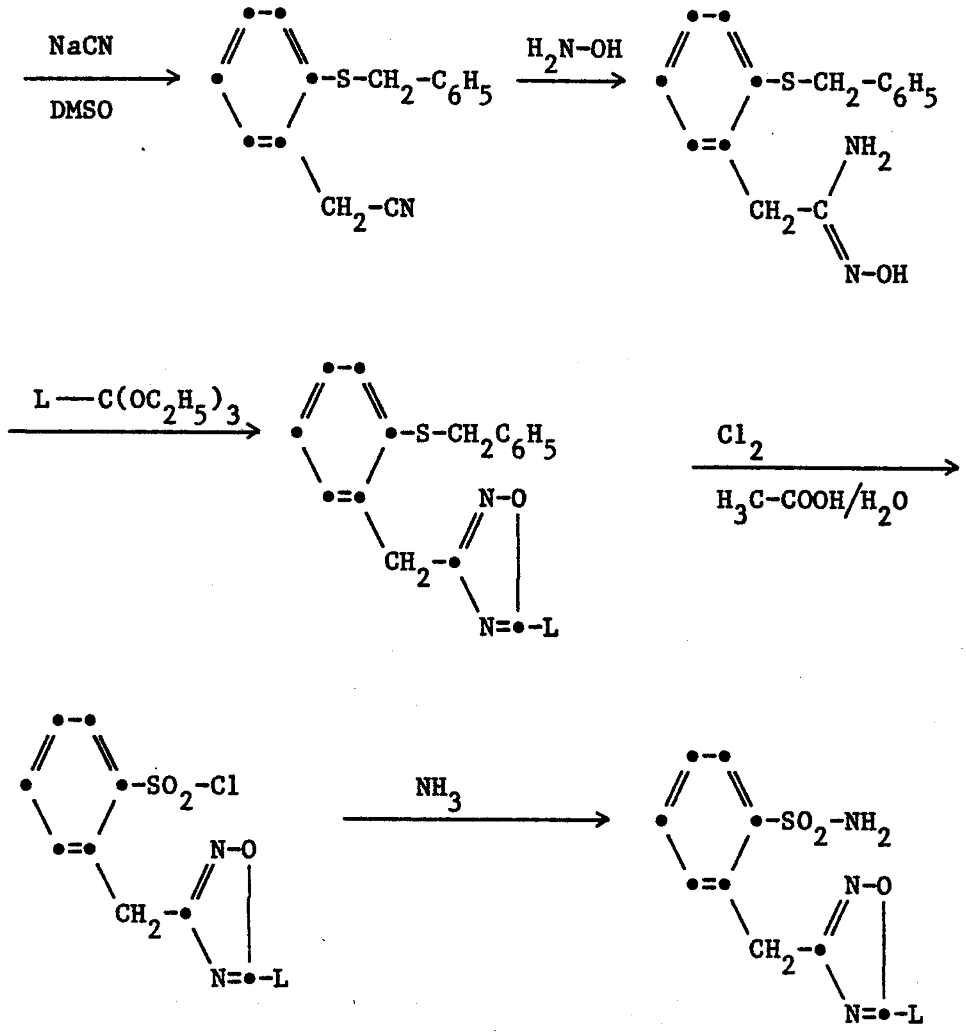
NaCN
DMSO
H2N-OH
L—C(OC2H5)3
Cl2
H3C-COOH/H2O
NH3

Schema 4:

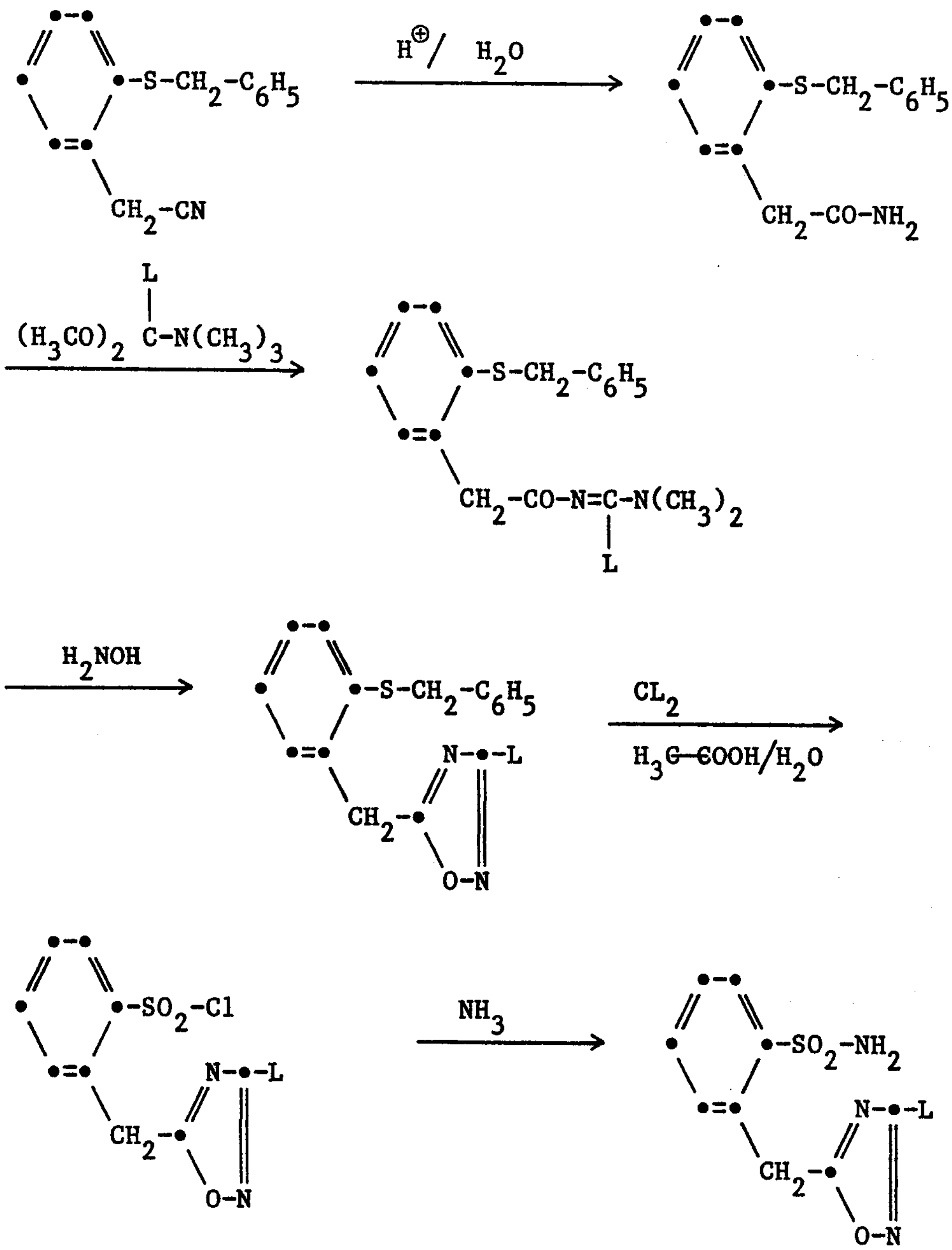


Schema 5:

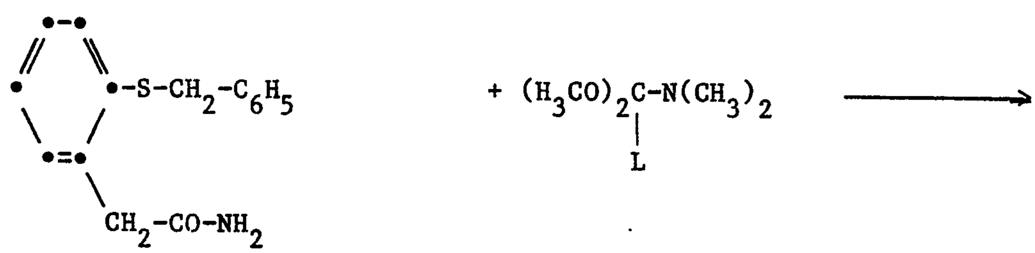

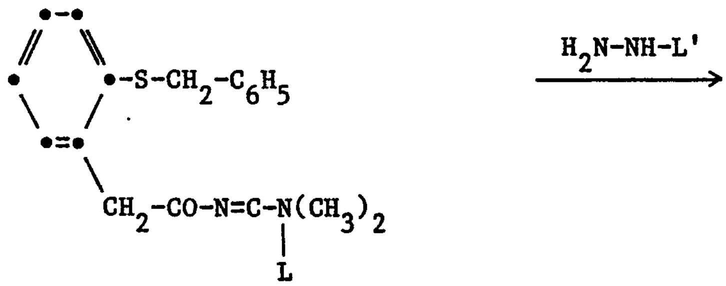


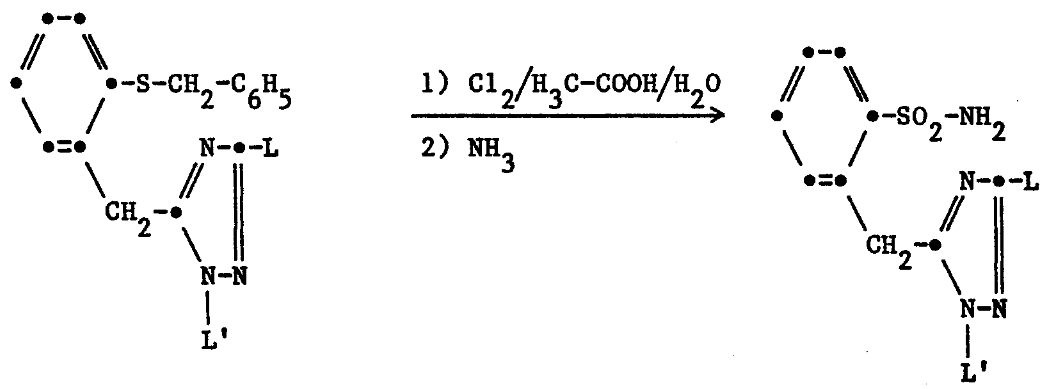


Schema 6:

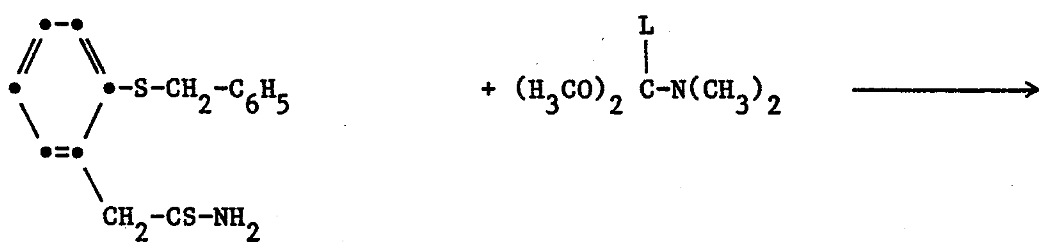

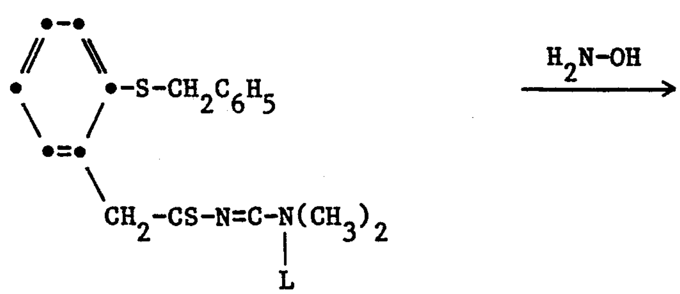

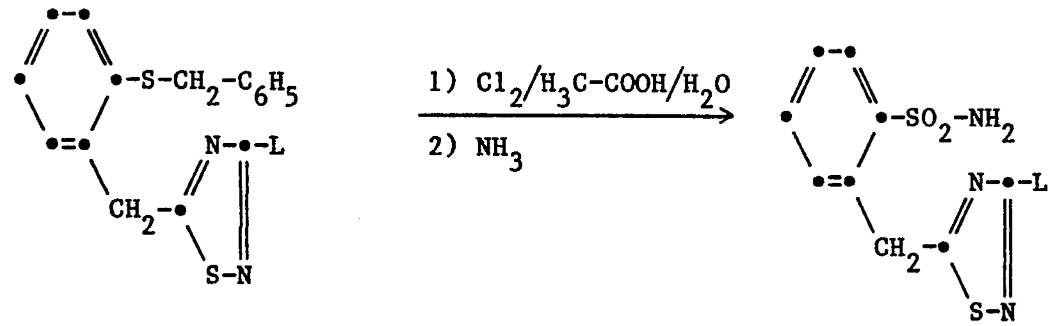
1) $Cl_2/H_3C-COOH/H_2O$
2) $NH_3$

Schema 7:

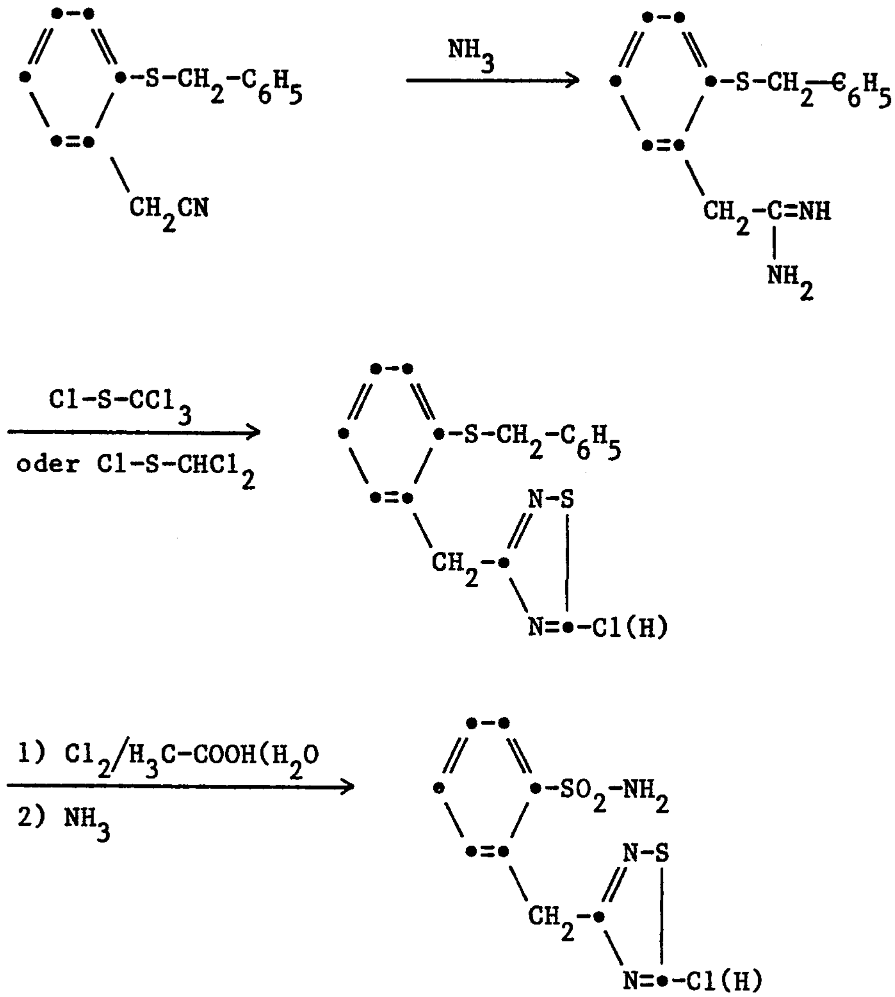
$NH_3$
$Cl-S-CCl_3$
oder $Cl-S-CHCl_2$
1) $Cl_2/H_3C-COOH(H_2O$
2) $NH_3$

Schema 8

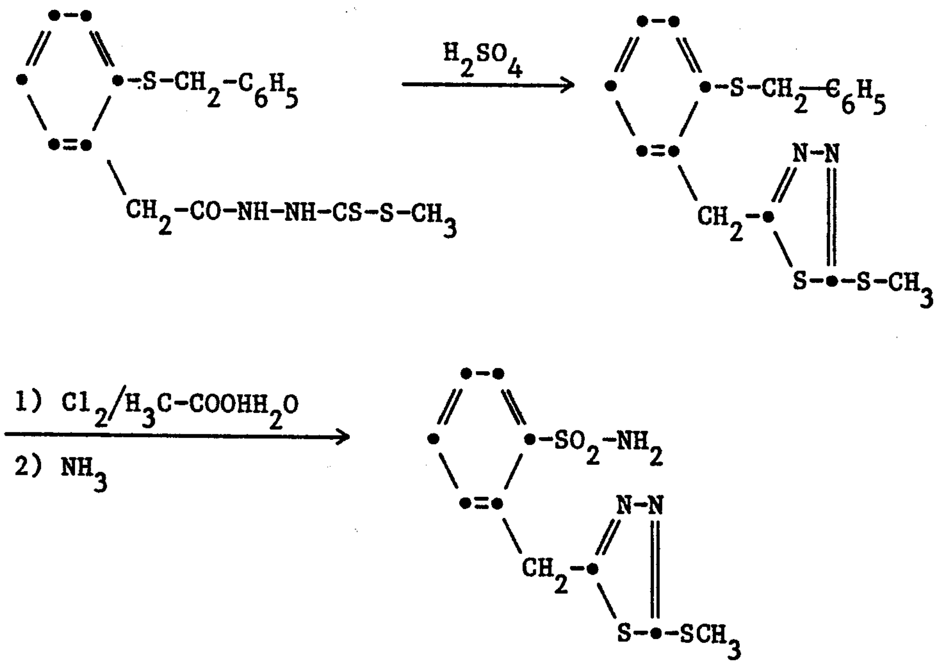


Schema 9:

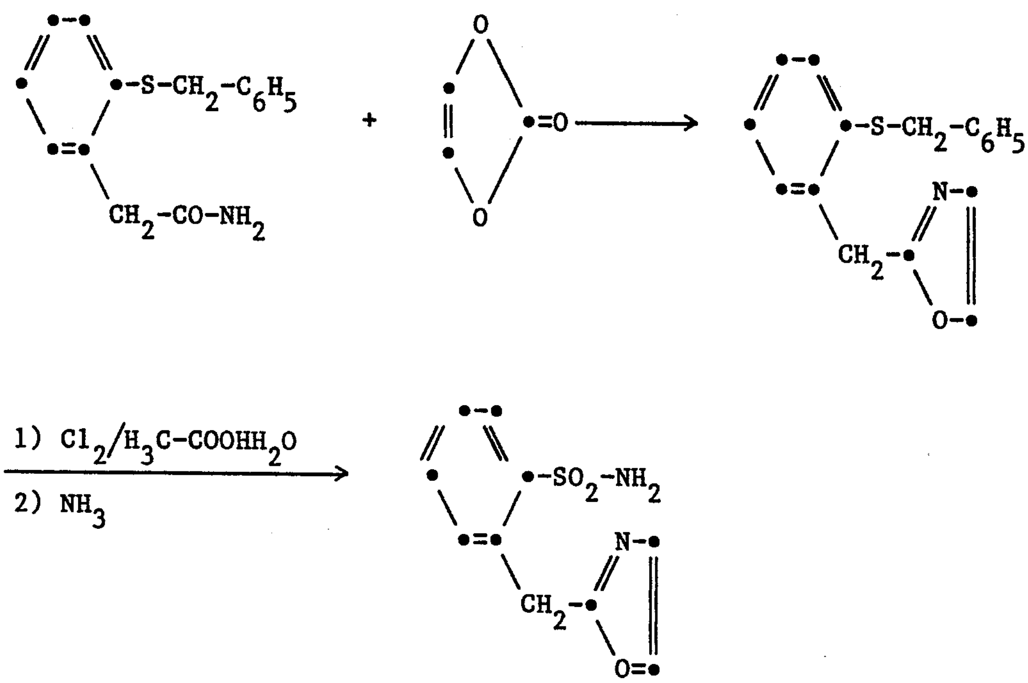

Schema 10:

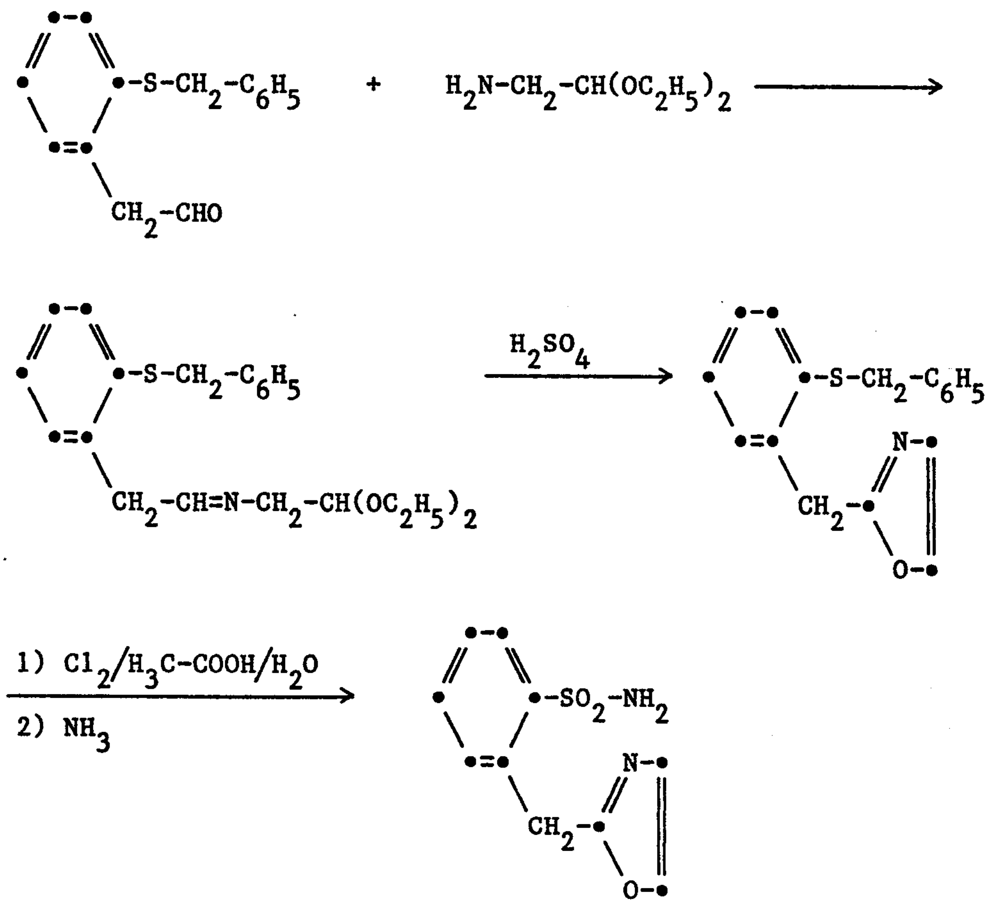


Schema 11:

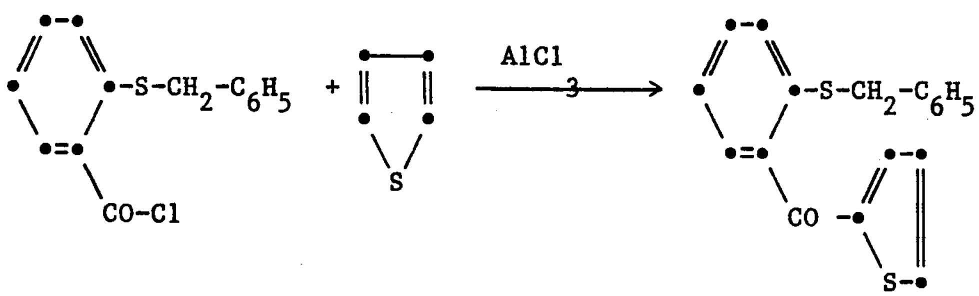

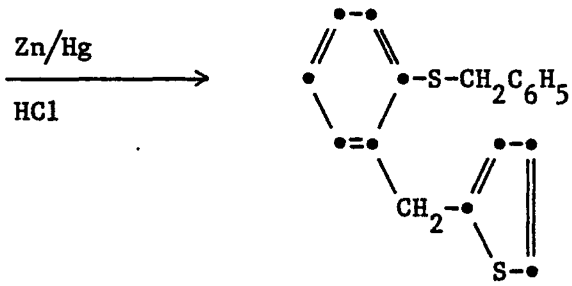


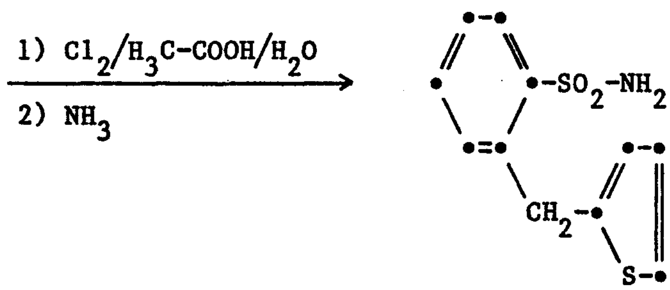


Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Aether, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsorglichen Massnahme.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter

bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzte Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzgut die Fraktionen $C_8$ bis $C_{12}$, wie z.B. Xylolgemische oder substituierte Naphtahline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen

wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und

einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl-oder

niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die pestiziden Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate:

| | |
|---|---|
| Aktiver Wirkstoff: | 1 bis 20 %, bevorzugt 5 bis 10 % |
| oberflächenaktive Mittel: | 5 bis 30 %, vorzugsweise 10 bis 20 % |
| flüssiges Trägermittel: | 50 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspensions-Konzentrate:

| | |
|---|---|
| Aktiver Wirkstoff: | 5 bis 75 %, vorzugsweise 10 bis 50 % |
| Wasser: | 94 bis 25 %, vorzugsweise 88 bis 30 % |
| oberflächenaktives Mittel: | 1 bis 40 %, vorzugsweise 2 bis 30 % |

Benetzbares Pulver:

| | | |
|---|---|---|
| Aktiver Wirkstoff: | 0,5 bis 90 %, | vorzugsweise 1 bis 80 % |
| oberflächenaktives Mittel: | 0,5 bis 20 %, | vorzugsweise 1 bis 15 % |
| festes Trägermittel: | 5 bis 95 %, | vorzugsweise 5 bis 90 % |

Granulate:

| | | |
|---|---|---|
| Aktiver Wirkstoff: | 0,5 bis 30 %, | vorzugsweise 3 bis 15 % |
| festes Trägermittel: | 99,5 bis 70 %, | vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele:

Beispiel H 1: 4,5-Dihydro-2-(2-sulfamoylphenyl-methylthio)-thiazol (Verb. Nr. 1.1)

Eine Suspension von 6,2 g 97 %igem 4,5-Dihydro-2-mercapto-thiazol in 50 ml Acetonitril wird unter Kühlung mit 7,9 g 97 %igem 1,5-Diaza bicyclo(5,4,0)undec-5-en versetzt. Dabei entsteht eine gelbe Lösung. Dieser Lösung setzt man 10,3 g 2-Chlormethyl-benzolsulfonamid zu und rührt für 1 Stunde bei 20 bis 25°C. Anschliessend wird die Reaktionslösung mit ca. 1 l Wasser verdünnt. Das ausgefallene Produkt wird abgetrennt und getrocknet. Man erhält so 11,9 g (76 % d.Th.) 4,5-Dihydro-2-(2-sulfamoylphenyl-methylthio)-thiazol, Smp. 123-125°C.

Beispiel H 2: 2-(2-Sulfamoylphenyl-methylthio)-pyrimidin (Verb. Nr. 1.5).

Eine Suspension von 5,7 g 98 %igem 2-Mercaptopyrimidin in 50 ml Acetonitril wird unter Kühlung mit 7,9 g 97 %igem 1,5-Diazabi cyclo(5,4,0)undec-5-en versetzt. Dabei entsteht eine gelbliche Lösung, die mit 10,3 g 2-Chlormethyl-benzolsulfonamid versetzt wird. Die Reaktionslösung wird für 70 Stunden bei 20-22°C gerührt und anschliessend mit 1 l Wasser verdünnt. Das ausgefallene Produkt wird abgetrennt und getrocknet. Man erhält so 12,0 g (85 % d.Th.) 2-(2-Sulfamoylphenyl-methylthio)-pyrimidin, Smp. 128-130°C.

Beispiel H 3: N-[2-(4,5-Dihydro-thiazol-2-yl-methylthio)-phenylsulfonyl]-N'-(4-methoxy-6-methyl-pyrimidin-2-yl)-harnstoff (Verb. Nr. 2.1).

Eine Lösung von 5,3 g 4,5-Dihydro-2-(2-sulfamoylphenyl-methylthio)-thiazol und 4,8 g N-(4-Methoxy-6-methyl-pyrimidin-2-yl)-O-phenyl-carbamat in 50 ml Acetonitril wird unter Kühlung mit 2,8 g 1,5-Diazabicyclo(5,4,0)undec-5-en versetzt. Die entstehende gelbe Lösung wird für zwei Tage bei 20-25°C gerührt und anschliessend unter Kühlung mit 1,7 g Methansulfonsäure versetzt. Nach dem Einsetzen der Kristallisation wird das Gemisch mit 1 l Wasser verdünnt und das auskristallisierte Produkt abgetrennt, mit Aether gewaschen und getrocknet. Man erhält so 7,0 g (85 % d.Th.) N-[2-(4,5-Dihydro-thiazol-2-yl-thiomethyl)-phenylsulfonyl]-N'-(4-methoxy-6-methyl-pyrimidin-2-yl)-harnstoff, Smp. 175-175°C (Zers.)

Beispiel H 4: N-[2-(Pyrimidin-2-yl-methylthio)-phenylsulfonyl]-N'-(4-methoxy-6-methyl-1.3.5-triazin-2-yl)-harnstoff (Verb. Nr. 2.10).

Eine Lösung von 5,7 g 2-(2-Sulfamoylphenyl-methylthio)-pyrimidin und 5,2 g N-(4-Methoxy-6-methyl-1.3.5-triazin-2-yl)-O-phenyl-carbamat in 50 ml Acetonitril wird unter Kühlung mit 3,1 g 1,5-Diazadicyclo-(5,4,0)undec-5-en versetzt. Die entstehende gelbliche Lösung wird für 50 Stunden bei 20-25°C gerührt und dann mit 1,9 g Methansulfonsäure versetzt. Nach dem Einsetzen der Kristallisation wird das Gemisch mit 1 l Wasser verdünnt und das ausgefallene Produkt abgetrennt, mit Aether gewaschen und getrocknet. Man erhält so 7,5 g

(84 % d.Th.) N-[2-(Pyrimidin-2-yl-thiomethyl)-phenylsulfonyl]-N'-(4-methoxy-6-methyl-1.3.5-triazin-2-yl)-harnstoff, Smp. 196-198°C (Zers.).

In analoger Weise werden die in den angeschlossenen Tabellen aufgelisteten Zwischen- und Endprodukte hergestellt.

Tabelle 1:

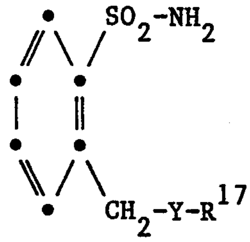

| Verbindung Nr. | Y | $R^{17}$ | Smp. [°C] |
|---|---|---|---|
| 1.1 | S | 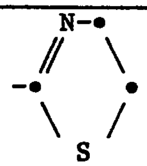 | 123-125 |
| 1.2 | S | 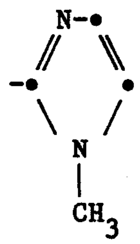 | 182-184 |
| 1.3 | S | 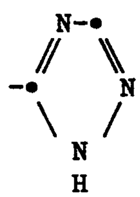 | 188-190 |
| 1.4 | S | 2-Pyridinyl | 121-123 |
| 1.4 | S | 2-Pyrimidinyl | 128-130 |
| 1.6 | S | 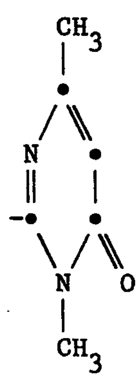 | 194-196 |
| 1.7 | O | 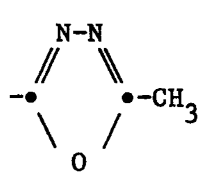 | |

Tabelle 2:

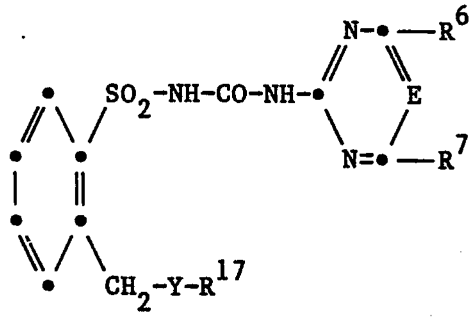

| Verb. Nr. | E | Y | $R^{17}$ | $R^6$ | $R^7$ | Smp. [°C] |
|---|---|---|---|---|---|---|
| 2.1 | CH | S | 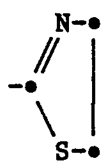 | $CH_3$ | $OCH_3$ | 174-175 (Zers.) |
| 2.2 | N | S | 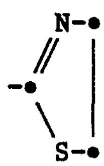 | $CH_3$ | $OCH_3$ | 177-179 (Zers.) |
| 2.3 | CH | S | 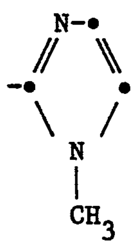 | $CH_3$ | $OCH_3$ | 190-192 (Zers.) |
| 2.4 | N | S | 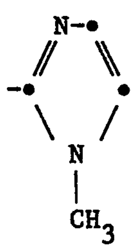 | $CH_3$ | $OCH_3$ | 180-182 (Zers.) |

Tabelle 2 (Fortsetzung)

| Verb. Nr. | E | Y | $R^{17}$ | $R^6$ | $R^7$ | Smp. [°C] |
|---|---|---|---|---|---|---|
| 2.5 | CH | S | 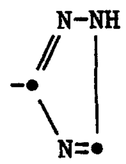 | $CH_3$ | $OCH_3$ | 217-218 (Zers.) |
| 2.6 | N | S | 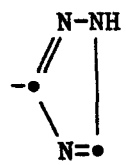 | $CH_3$ | $OCH_3$ | 189-191 (Zers.) |
| 2.7 | CH | S | 2-Pyridinyl | $CH_3$ | $OCH_3$ | 199-201 (Zers.) |
| 2.8 | CH | S | 2-Pyrimidinyl | $CH_3$ | $CH_3$ | 217-219 (Zers.) |
| 2.9 | CH | S | 2-Pyrimidinyl | $CH_3$ | $OCH_3$ | 211-213 (Zers.) |
| 2.10 | N | S | 2-Pyrimidinyl | $CH_3$ | $OCH_3$ | 196-198 (Zers.) |
| 2.11 | CH | S | 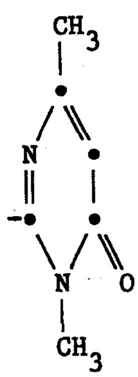 | $CH_3$ | $OCH_3$ | 226-228 (Zers.) |

Tabelle 2 (Fortsetzung)

| Verb. Nr. | E | Y | $R^{17}$ | $R^6$ | $R^7$ | Smp. [°C] |
|---|---|---|---|---|---|---|
| 2.12 | N | S | 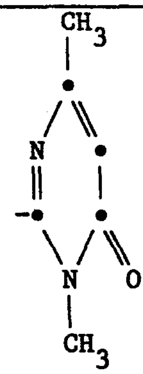 | $CH_3$ | $OCH_3$ | 203-205 (Zers.) |
| 2.13 | CH | O | 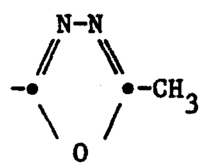 | $CH_3$ | $OCH_3$ | |
| 2.14 | N | O | 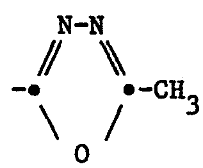 | $CH_3$ | $OCH_3$ | |
| 2.15 | CH | S | 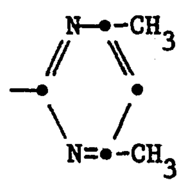 | $CH_3$ | $OCH_3$ | 217-219 (Zers.) |

Tabelle 2 (Fortsetzung)

| Verb. Nr. | E | Y | $R^{17}$ | $R^6$ | $R^7$ | Smp. [°C] |
|---|---|---|---|---|---|---|
| 2.16 | N | S | 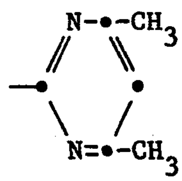 | $CH_3$ | $OCH_3$ | 188-190 (Zers.) |
| 2.17 | CH | S | 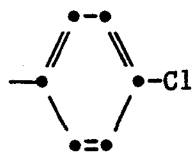 | $CH_3$ | $OCH_3$ | 217-219 (Zers.) |
| 2.18 | CH | S | 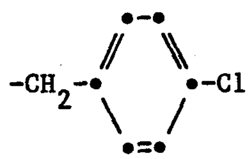 | $CH_3$ | $OCH_3$ | 142-145 |
| 2.19 | N | S | 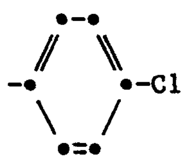 | $CH_3$ | $OCH_3$ | 202-204 |
| 2.20 | N | S | 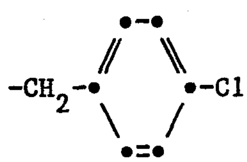 | $CH_3$ | $OCH_3$ | 170-173 |
| 2.21 | N | S | 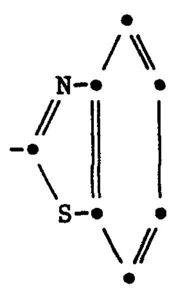 | $CH_3$ | $OCH_3$ | 198-199 |

Tabelle 2 (Fortsetzung)

| Verb. Nr. | E | Y | $R^{17}$ | $R^6$ | $R^7$ | Smp. [°C] |
|---|---|---|---|---|---|---|
| 2.22 | CH | S | 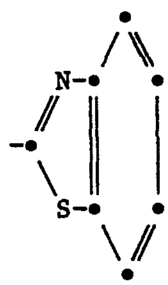 | $CH_3$ | $OCH_3$ | 212-214 (Zers.) |
| 2.23 | N | S | 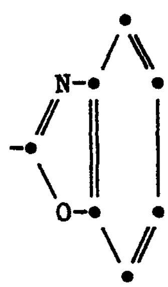 | $CH_3$ | $OCH_3$ | 198-199 (Zers.) |
| 2.24 | CH | S | 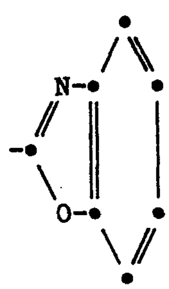 | $CH_3$ | $OCH_3$ | 198-199 (Zers.) |
| 2.25 | N | S | 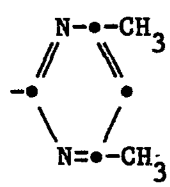 | $-N(CH_3)_2$ | $-OCH_3$ | 200-205 |
| 2.26 | N | S | 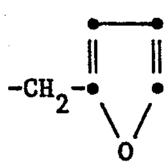 | $CH_3$ | $OCH_3$ | 128-129 |

Tabelle 2 (Fortsetzung)

| Verb. Nr. | E | Y | $R^{17}$ | $R^6$ | $R^7$ | Smp. [°C] |
|---|---|---|---|---|---|---|
| 2.27 | CH | S | 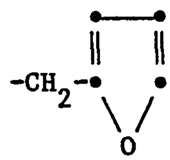 | $CH_3$ | $OCH_3$ | 141-143 |
| 2.28 | CH | S | 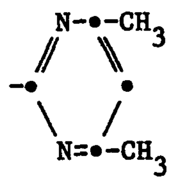 | $OCH_3$ | $OCH_3$ | 215-220 |
| 2.29 | CH | S | 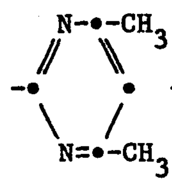 | $-CH_2-OCH_3$ | $OCH_3$ | 187-189 |
| 2.30 | CH | O | 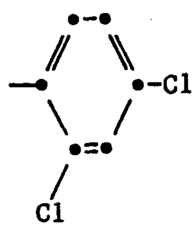 | $CH_3$ | $CH_3$ | 221-222 |
| 2.31 | N | O | 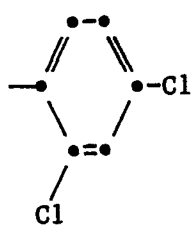 | $CH_3$ | $OCH_3$ | 195-196 |

Formulierungsbeispiele:

Beispiel F 1: Formulierungsbeispiele für Wirkstoffe der Formel I (% = Gewichtsprozent)

| a) Spritzpulver | a) | b) | c) |
| --- | --- | --- | --- |
| Wirkstoff | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6% | 6 % |
| Octylphenolpolyäthylenglykoläther /7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
| --- | --- | --- |
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10% |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40 % | 5 % |
| Aethylenglykol | 10 % | 10 % |
| Nonylphenolpolyäthylenglykoläther (15 MOl AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |

| | | |
|---|---|---|
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (78 Mol AeO) | 3 % |
| Wasser | 91 % |

Biologische Beispiele:

Beispiel B 1: Herbizidwirkung vor dem Auflaufen der Pflanzen

Kunststofftöpfe werden mit expandiertem Vermiculit (Dichte: 0,135 g/$cm^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 xLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5.Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem folgenden Massstab bewertet:

1 : Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:
Konzentration der Wirkstoffemulsion: 70,8 ppm

| Testpflanze / Wirkstoff Nr | Nasturtium | Stellaria | Agrostis | Digitaria |
|---|---|---|---|---|
| 2.1 | 1 | 1 | 1 | 1 |
| 2.2 | 1 | 1 | 1 | 1 |
| 2.3 | 1 | 2 | 1 | 3 |
| 2.4 | 2 | 2 | 2 | 5 |
| 2.5 | 2 | 2 | 2 | 3 |
| 2.6 | 4 | 4 | 4 | 7 |
| 2.7 | 1 | 1 | 1 | 1 |
| 2.9 | 1 | 1 | 1 | 1 |
| 2.10 | 1 | 1 | 1 | 2 |
| 2.11 | 2 | 2 | 2 | 3 |
| 2.12 | 2 | 7 | 3 | 8 |
| 2.15 | 2 | 2 | 2 | 2 |
| 2.16 | 1 | 2 | 2 | 2 |
| 2.17 | 2 | 2 | 2 | 2 |
| 2.18 | 1 | 1 | 1 | 2 |
| 2.19 | 1 | 3 | 3 | 6 |
| 2.20 | 2 | 2 | 3 | 3 |

| Testpflanze Wirkstoff Nr | Nasturtium | Stellaria | Agrostis | Digitaria |
|---|---|---|---|---|
| 2.21 | 2 | 2 | 2 | 3 |
| 2.22 | 2 | 4 | 1 | 5 |
| 2.23 | 2 | 2 | 2 | 2 |
| 2.24 | 1 | 1 | 1 | 2 |
| 2.25 | 1 | 2 | 1 | 3 |
| 2.26 | 1 | 1 | 1 | 3 |
| 2.27 | 2 | 2 | 1 | 2 |
| 2.28 | 3 | 2 | 2 | 3 |
| 2.29 | 3 | 2 | 2 | 4 |
| 2.31 | 2 | 2 | 5 | 3 |

Beispiel B 2: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 600 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehanelten Kontrollpflanzen), ohne dass dabei die Versuchspflanzen geschädigt wurden.

Beispiel B 3: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel B 4: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel B 5: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

Patentansprüche

1. Substituierte N-Phenylsulfonyl-N'-pyrimidinyl- und -triazinylharnstoffe der Formel

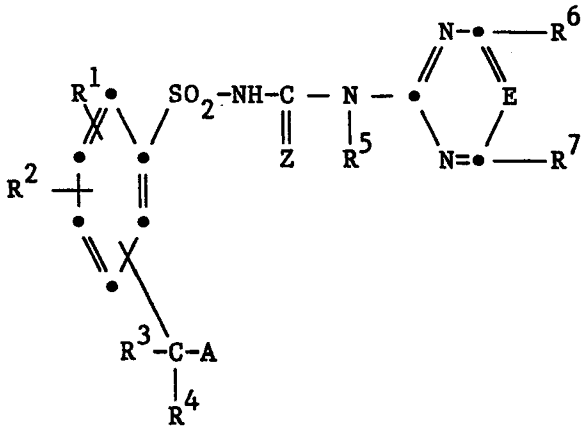

(I),

worin

$R^1$ Wasserstoff, Halogen, Nitro, Cyan, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfonyl, $C_1$-$C_4$-Alkylsulfinyl, $-CO-R^8$, $-NR^9R^{10}$, $-CO-NR^{11}R^{12}$ oder $-SO_2-NR^{13}R^{14}$,

$R^2$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^3$ Wasserstoff, $C_1$-$C_4$-Alkyl, $C_1$-$C_4$-Halogenalkyl oder Cyan,

$R^4$ Wasserstoff oder $C_1$-$C_4$-Alkyl,

$R^5$ Wasserstoff, $C_1$-$C_4$-Alkyl oder $C_1$-$C_4$-Alkoxy,

$R^6$ und $R^7$ unabhängig voneinander Wasserstoff, Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_1$-$C_4$-Halogenalkylthio, $C_2$-$C_4$-Alkoxyalkyl, $C_3$-$C_6$-Cycloalkyl oder $-NR^{15}R^{16}$,

$R^8$ Wasserstoff, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_2$-$C_4$-Alkoxyalkoxy,

$R^9$, $R^{10}$, $R^{11}$, $R^{12}$, $R^{13}$, $R^{14}$, $R^{15}$ und $R^{16}$ unabhängig voneinander Wasserstoff oder $C_1$-$C_4$-Alkyl,

A einen Rest $-Y-(CH_2)_n-R^{17}$ oder

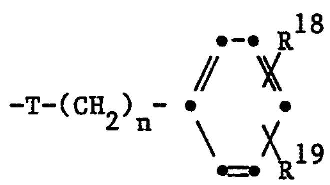

$R^{17}$ einen 5- bis 6-gliedrigen ungesättigen heterocyclischen Rest,

$R^{18}$ und $R^{19}$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, Nitro oder Cyan,

E Stickstoff oder -CH=,

Y Sauerstoff, Schwefel oder eine direkte Bindung,

T und Z unabhängig voneinander Sauerstoff oder Schwefel und

n Null oder eins bedeuten, sowie Salze dieser Verbindungen.

2. Verbindungen gemäss Anspruch 1, dadurch gekennzeichnet, dass Z für Sauerstoff steht.

3. Verbindungen gemäss Anspruch 1, dadurch gekennzeichnet, dass $R^1$ für Wasserstoff steht.

4. Verbindungen gemäss Anspruch 1, dadurch gekennzeichnet, dass $R^2$ für Wasserstoff steht.

5. Verbindungen gemäss Anspruch 1, dadurch gekennzeichnet, dass $R^3$ für Wasserstoff steht.

6. Verbindungen gemäss Anspruch 1, dadurch gekennzeichnet, dass $R^4$ für Wasserstoff steht.

7. Verbindungen gemäss Anspruch 1, dadurch gekennzeichnet, dass $R^5$ für Wasserstoff steht.

8. Verbindungen gemäss Anspruch 1, dadurch gekennzeichnet, dass $R^6$ und $R^7$ unabhängig voneinander für Halogen, $C_1$-$C_4$-Alkyl, $C_1$-$C_4$-Alkoxy, Di-$C_1$-$C_4$-alkylamino der $C_1$-$C_4$-Halogenalkoxy stehen und zusammen höchstens 4 Kohlenstoffatome enthalten.

9. Verbindungen gemäss Anspruch 1, dadurch gekennzeichnet, dass A für die Gruppe $-Y-(CH_2)_n-R^{17}$ steht.

10. Verbindungen gemäss Anspruch 9, dadurch gekennzeichnet, dass n für Null steht.

11. Verbindungen gemäss Anspruch 10, dadurch gekennzeichnet, dass A für den Rest $-S-R^{17}$ steht.

12. Verbindungen gemäss Anspruch 11, dadurch gekennzeichnet, dass $-S-R^{17}$ für einen Rest steht, ausgewählt aus der Gruppe 4,5-Dihydrothiazol-2-ylthio, 3-Methylimidazol-2-ylthio, 1,3,4-Triazol-2-ylthio, Pyridin-2-ylthio, Pyrimidin-2-ylthio oder 3H-2,6-Dimethyl-3-oxo-pyrimidin-2-ylthio.

13. Verbindungen gemäss Anspruch 1, dadurch gekennzeichnet, dass Z für Sauerstoff steht, $R^1$, $R^2$, $R^3$, $R^4$ und $R^5$ Wasserstoff bedeuten und $R^6$ und $R^7$ unabhängig voneinander für Halogen, $C_1$-$C_4$-Alkyl, $C_1$-$C_4$-Alkoxy, Di-$C_1$-$C_4$-alkylamino oder $C_1$-$C_4$-Halogenalkoxy stehen und zusammen höchstens 4 Kohlenstoffatome enthalten.

14. Verbindungen gemäss Anspruch 1, dadurch gekennzeichnet, dass Z für Sauerstoff steht, $R^1$, $R^2$, $R^3$, $R^4$ und $R^5$ Wasserstoff bedeuten, $R^6$ und $R^7$ unabhängig voneinander für Halogen, $C_1$-$C_4$-Alkyl, $C_1$-$C_4$-Alkoxy, Di-$C_1$-$C_4$-alkylamino oder $C_1$-$C_4$-Halogenalkoxy stehen und zusammen höchstens 4 Kohlenstoffatome enthalten und A für den Rest -S-$R^{17}$ steht.

15. Verbindungen gemäss Anspruch 1, dadurch gekennzeichnet, dass Z für Sauerstoff steht, $R^1$, $R^2$, $R^3$, $R^4$ und $R^5$ Wasserstoff bedeuten, $R^6$ und $R^7$ unabhängig voneinander für Halogen, $C_1$-$C_4$-Alkyl, $C_1$-$C_4$-Alkoxy, Di-$C_1$-$C_4$-alkylamino oder $C_1$-$C_4$-Halogenalkoxy stehen und zusammen höchstens 4 Kohlenstoffatome enthalten, n Null bedeutet und A für einen Rest steht, ausgewählt aus der Gruppe 4,5-Dihydrothiazol-2-ylthio, 3-Methylimidazol-2-ylthio, 1,3,4-Triazol-2-ylthio, Pyridin-2-ylthio, Pyrimidin-2-ylthio oder 3H-2,6-Dimethyl-3-oxopyrimidin-2-ylthio.

16. Eine Verbindung gemäss Anspruch 1, dadurch gekennzeichnet, dass sie ausgewählt ist aus der Gruppe N-[2-(4,5-Dihydrothiazol-2-ylthiomethyl)-phenylsulfonyl]-N'-(4-methoxy-6-methyl-pyrimidin-2-yl)-harnstoff, N-[2-(Pyridin-2-ylthiomethyl)-phenylsulfonyl]-N'-(4-methoxy-6-methyl-1.3.5-triazin-2-yl)-harnstoff oder N-[2-(Pyrimidin-2-ylthiomethyl)-phenylsulfonyl]-N'-(4-methoxy-6-methyl-pyrimidin-2-yl)-harnstoff.

17. Verfahren zur Herstellung der Verbindungen der Formel I gemäss Anspruch 1, dadurch gekennzeichnet, dass man ein substituiertes Phenylsulfonamid der Formel II

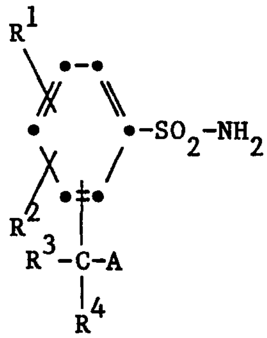

(II),

worin $R^1$, $R^2$, $R^3$, $R^4$, und A die unter Formel I in Anspruch 1 gegebene Bedeutung haben, in Gegenwart einer Base mit einem N-Pyrimidinyl- oder -triazinylcabamat der Formel III

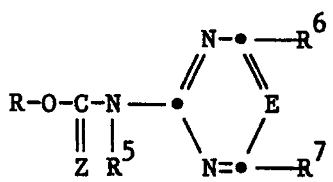

(III)

worin E, $R^5$, $R^6$, $R^7$ und Z die unter Formel I in Anspruch 1 gegebene Bedeutung haben und R für Phenyl, Alkyl oder substituiertes Phenyl steht, umsetzt und gegebenenfalls in die Salze überführt.

18. Verfahren zur Herstellung der Verbindungen der Formel I gemäss Anspruch 1, dadurch gekennzeichnet, dass man ein substituiertes N-Phenylsulfonylcarbamat der Formel IV

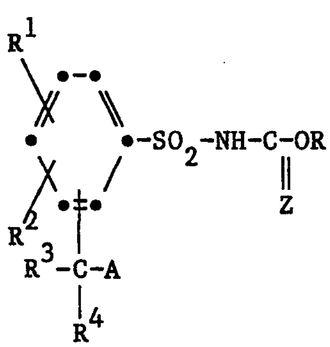

(IV),

worin A, $R^1$, $R^2$, $R^3$, $R^4$ , Y, n und Z die unter Formel I in Anspruch 1 gegebene Bedeutung haben und R für Phenyl, Alkyl oder substituiertes Phenyl steht, mit einem Aminopyrimidin oder -triazin der Formel V

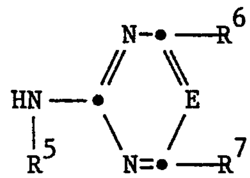

(V),

worin E, $R^5$, $R^6$ und $R^7$ die unter Formel I in Anspruch 1 gegebene Bedeutung haben, umsetzt und gegebenenfalls in die Salze überführt.

19. Verfahren zur Herstellung der Verbindungen der Formel I, in denen Y keine direkte Bindung bedeutet, gemäss Anspruch 1, dadurch gekennzeichnet, dass man entweder einen Sulfonylharnstoff der Formel VI

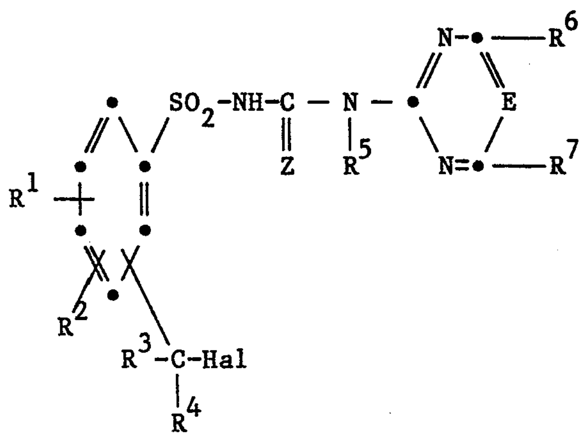

(VI),

worin $R^1$, $R^2$, $R^3$, $R^4$, $R^5$, $R^6$, $R^7$, E und Z die unter Formel I in Anspruch 1 gegebene Bedeutung haben und Hal für Halogen steht, in Gegenwart einer Base mit einem Alkohol oder einem Mercaptan der Formeln VII oder VIII

$H-Y-(CH_2)_n-R^{17}$ (VII) , H - T - $(CH_2)_n$ - 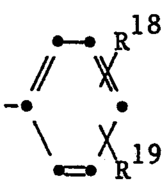 (VIII)

worin $R^{17}$, $R^{18}$, $R^{19}$, n und T die unter Formel I gegebene Bedeutung haben, und Y für Sauerstoff oder Schwefel steht, umsetzt, oder indem man eine Verbindung der Formel IX

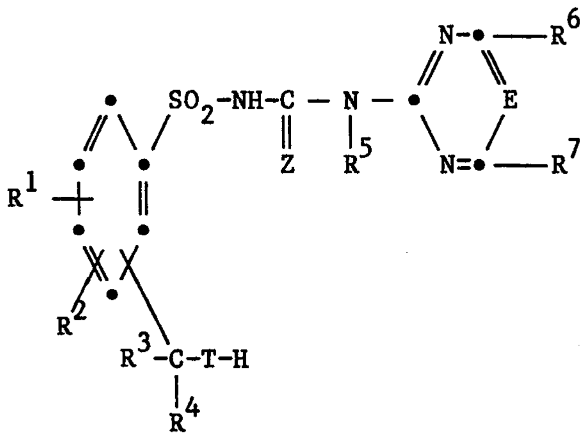

(IX),

worin $R^1$, $R^2$, $R^3$, $R^4$, $R^5$, $R^6$, $R^7$, E, T und Z die unter Formel I gegebene Bedeutung haben, in Gegenwart einer Base mit einer Halogenverbindung der Formeln X oder XI

$Hal—(CH_2)_n—R^{17}$ , Hal-$(CH_2)$ - 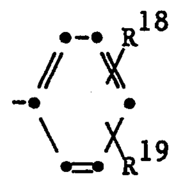

(X) (XI)

worin $R^{17}$, $R^{18}$, $R^{19}$ und n die unter Formel I gegebene Bedeutung haben und Hal für Halogen, vorzugsweise Brom oder Chlor steht, umsetzt, und gegebenenfalls in ein Salz überführt.

20. Verfahren zur Herstellung von Additionssalzen der Formel I gemäss einem der Ansprüche 17 bis 19, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

21. Ein herbizides und den Pflanzenwuchs hemmendes Mittel, dadurch gekennzeichnet, dass es neben Träger- und/oder anderen Zuschlagstoffen als Wirkstoff mindestens einen substituierten N-Phenylsulfonyl-N'-pyrimidinyl- oder -triazinyl-harnstoff der Formel I, Anspruch 1, enthält.

22. Die Verwendung der Wirkstoffe der Formel I, gemäss Anspruch 1, oder sie enthaltender Mittel zur Bekämpfung unerwünschten Pflanzenwachstums.

23. Die Verwendung der Wirkstoffe der Formel I, gemäss Anspruch 1, oder sie enthaltender Mittel zur Hemmung des Pflanzenwachstums.

24. Die Verwendung der Wirkstoffe der Formel I, gemäss Anspruch 1, oder sie enthaltender Mittel zur Beeinflussung des Pflanzenwachstums zum Zweck der Ertragssteigerung.

25. Die Verwendung der Wirkstoffe der Formel I, oder sie enthaltender Mittel, gemäss Anspruch 22, zur selektiven pre- oder postemergenten Bekämpfung von Unkräutern in Nutzpflanzenkulturen.

26. Die Verwendung der Wirkstoffe gemäss Anspruch 23, zur Unterdrückung des Pflanzenwachstums über das 2-Blattstadium hinaus, dadurch gekennzeichnet, dass die Wirkstoffe preemergent angewendet werden.

27. Substituierte Phenylsulfonamide der Formel II

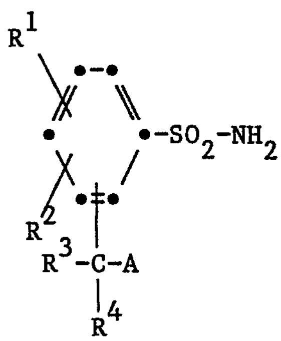

(II),

worin $R^1$, $R^2$, $R^3$, $R^4$, und A die unter Formel I im Anspruch 1 angegebene Bedeutung haben.

FO 7.5 CW/eg*